(19) Europäisches Patentamt / European Patent Office / Office européen des brevets

(11) **EP 4 549 618 A1**

(12) **EUROPEAN PATENT APPLICATION**

(43) Date of publication:
**07.05.2025  Bulletin 2025/19**

(21) Application number: **24207979.6**

(22) Date of filing: **22.10.2024**

(51) International Patent Classification (IPC):
**C25B 1/04** $^{(2021.01)}$    **C25B 15/08** $^{(2006.01)}$
**C01B 4/00** $^{(2006.01)}$    **C01C 1/04** $^{(2006.01)}$
**C07B 59/00** $^{(2006.01)}$    **C07C 209/48** $^{(2006.01)}$

(52) Cooperative Patent Classification (CPC):
(C-Sets available)
**C25B 1/04; C01B 4/00; C01C 1/0405;
C07B 59/001; C07C 209/48; C25B 15/081;**
C07B 2200/05; C07C 2601/14          (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **31.10.2023  EP 23206973**

(71) Applicant: **BASF SE**
**67056 Ludwigshafen am Rhein (DE)**

(72) Inventors:
• **VOSSEN, Marcus**
**67117 Limburgerhof (DE)**
• **ERNST, Martin**
**67056 Ludwigshafen am Rhein (DE)**

• **STOCK, Christoph**
**67056 Ludwigshafen am Rhein (DE)**
• **HARNHAUSEN, Sina**
**67056 Ludwigshafen am Rhein (DE)**
• **XIONG, Jiawen**
**67056 Ludwigshafen am Rhein (DE)**
• **HUEFFER, Stephan**
**67063 Ludwigshafen (DE)**
• **KRUEGER, Marco**
**67056 Ludwigshafen am Rhein (DE)**
• **WEISS, Thomas**
**67056 Ludwigshafen am Rhein (DE)**

(74) Representative: **BASF IP Association**
**BASF SE**
**GBI - Z078**
**67056 Ludwigshafen (DE)**

(54) **PROCESS FOR MAKING AMINES FROM NITRILE COMPOUNDS USING HYDROGEN HAVING LOW DEUTERIUM CONTENT PRODUCED WITH NON-FOSSIL ENERGY**

(57)    A process for the preparation of amines comprising the following steps:
(a) providing hydrogen with a molar share of deuterium $\leq$ 100 ppm, preferably in the range of from 10 to 95 ppm, more preferably in the range of from 15 to 90 ppm, most preferably in the range of from 20 to 80 ppm, especially in the range of from 30 to 75 ppm, based on the total hydrogen content, by electrolysis of water using electrical power generated at least in part from non-fossil energy,
(b) reacting the hydrogen from step (a) with nitrogen to form ammonia,
(c) reacting the ammonia from step (b) with a nitrile compound or hydrogen cyanide (I)

$$R\text{-}CN \qquad (I)$$

in the presence of hydrogen from step (a) to form the corresponding amine (II)

$$RCH_2\text{-}NH_2 \qquad (II).$$

EP 4 549 618 A1

(52) Cooperative Patent Classification (CPC): (Cont.)

C-Sets
**C07C 209/48, C07C 211/36**

**Description**

The present invention relates to a process for making amines having a lower deuterium content, based on the total hydrogen content, compared to amines generated by reaction with hydrogen produced from petrochemical processes using natural gas, condensate or petroleum oil, the amines obtained thereby as well as to their use. More specifically, the present invention relates to a process for making amines from nitrile compounds using hydrogen having low deuterium content produced from non-fossil electrical energy, the amines obtained thereby as well as to the use of the molar share of deuterium in the hydrogen bound in the amines for tracing the origin of the hydrogen.

In the preparation of amines from nitrile compounds, ammonia is a key precursor. In the preparation of amines from nitriles via hydrogenation, ammonia is a used to increase the yield and or the selectivity of the reaction. Since the development of the Haber-Bosch process for the preparation of ammonia, the vast majority of ammonia is manufactured by the direct synthesis from hydrogen and nitrogen in the presence of a catalyst, especially an iron-containing catalyst. Special care needs to be taken with the provision of the starting materials hydrogen and nitrogen. They should exhibit a high purity and be substantially free from catalyst poisoning agents such as carbon monoxide and sulfur compounds such as $H_2S$ and $SO_2$. In modern processes, a significant amount of the hydrogen is provided by steam reforming, thus, from natural gas.

However, the petrochemical steam reforming process has its negative impacts with regard to its carbon footprint including the consumption of a lot of fossil-based natural resources and energy.

It is therefore an object of the present invention to provide environmentally friendly amines in an environmentally friendly process for making the same, that process using as little fossil-based energy as possible.

The object is achieved by a process for the preparation of amines, wherein said process comprises the following steps:

(a) providing hydrogen with a molar share of deuterium $\leq$ 100 ppm, preferably in the range of from 10 to 95 ppm, more preferably in the range of from 15 to 90 ppm, most preferably in the range of from 20 to 80 ppm, especially in the range of from 30 to 75 ppm, based on the total hydrogen content, by electrolysis of water using electrical power generated at least in part from non-fossil energy,
(b) reacting the hydrogen from step (a) with nitrogen to form ammonia,
(c) reacting the ammonia from step (b) with a nitrile compound or hydrogen cyanide (I)

R-CN              (I)

in the presence of hydrogen from step (a) to form the corresponding amine (II)

$RCH_2-NH_2$              (II).

R is in general an aliphatic, heteroaliphatic (i. e. containing hetero atoms in the aliphatic chain), araliphatic, cycloaliphatic or heterocycloaliphatic residue having 1 to 23 carbon atoms.

In a further embodiment of the present invention, the object is achieved by amines, wherein the molar share of deuterium in the $-CH_2NH_2$ group or groups formed in step (c) is $\leq$ 100 ppm, preferably in the range of from 10 to 95 ppm, more preferably in the range of from 15 to 90 ppm, most preferably in the range of from 20 to 80 ppm, especially in the range of from 30 to 75 ppm based on the total hydrogen content of said $-CH_2NH_2$ group or groups.

It is important that the origin of the hydrogen and downstream compounds obtained by clean energy can be tracked in a reliable way. This is especially important to ensure that:

• Hydrogen and downstream compounds have been produced in accordance with sustainability criteria.
• Renewable attributes aren't subject to double counting.

Companies are placing increasing importance on sourcing green energy. Because of this, tracking systems have to be developed for the origin of the energy used in the preparation of hydrogen and downstream compounds.

This object is also achieved by use of the molar share of deuterium in hydrogen bound in downstream compounds based on hydrogen for tracing the origin, especially the energetic origin, of the hydrogen bound in the downstream compounds based on hydrogen, wherein the compounds are amines and a process for tracing the origin, especially the energetic origin, of hydrogen bound in downstream compounds based on hydrogen by determining the molar share of deuterium in the hydrogen bound in said downstream compounds. Methods for determination of the molar share of deuterium in hydrogen and in downstream compounds based on hydrogen are known to a person skilled in the art and include mass spectrometry and NMR technologies.

A further environmental benefit of the environmentally friendly amines according to the present invention is their use in carbon capturing processes, since the amines according to the present invention are produced using as little fossil-based energy as possible, ideally no fossil-based energy, and do therefore only add as little as possible, ideally nothing, to $CO_2$ emission.

A further embodiment of the present invention is therefore the use of the amines according to the present invention as liquid or solid $CO_2$ absorbents in $CO_2$ capturing processes.

Specific examples of amines usable with preference are:

i) 2-aminoethoxyethanol (ADEG), 2-aminoethanol (monoethanolamine), 2-(methylamino)ethanol, 2-(ethylamino) ethanol, 2-(n-butylamino)ethanol, 2-amino-2-methylpropanol, N-(2-aminoethyl)piperazine, methyldiethanolamine, ethyldiethanolamine, dimethylaminopropanol, tert-butylaminoethoxyethanol (TBAEE), methoxyethoxyethyl-tert-butylamine, 2-amino-2-methylpropanol, diisopropanolamine (DIPA or DIPOA);

ii) 3-methylaminopropylamine, ethylenediamine, diethylenetriamine, triethylenetetramine, 2,2-dimethyl-1,3-diaminopropane, hexamethylenediamine, 1,4-diaminobutane, 3,3-iminobispropylamine, tris(2-aminoethyl)amine, bis(3-dimethylaminopropyl)amine and tetramethylhexamethylenediamine;

iii) piperazine, 2-methylpiperazine, N-methylpiperazine, 1-hydroxyethylpiperazine, 1,4-bishydroxyethylpiperazine, 4-hydroxyethylpiperidine, homopiperazine, piperidine, 2-hydroxyethylpiperidine, triethylendiamine (TEDA) and morpholine; and

iv) mixtures thereof.

The molar share of deuterium in hydrogen and downstream compounds based on hydrogen is given in the present application in ppm, based on the total hydrogen content, which is the mol-ppm content of deuterium, based on the total hydrogen content (in hydrogen or in the compounds discussed, respectively).

The deuterium content of hydrogen and downstream compounds based on hydrogen is given in the present application in atom-ppm based on the total molar hydrogen content (total atoms of protium $^1H$ and deuterium $^2H$). The terms "deuterium content" and "molar share of deuterium" are used synonymously throughout the application.

In physical organic chemistry, a kinetic isotope effect is the change in the reaction rate of a chemical reaction when one of the atoms in the reactants is replaced by one of its isotopes. Formally, it is the ratio of rate constants $k_L / k_H$ for the reactions involving the light ($k_L$) and the heavy ($k_H$) isotopically substituted reactants (isotopologues). This change in reaction rate is a quantum mechanical effect that primarily results from heavier isotopologues having lower vibrational frequencies compared to their lighter counterparts. In most cases, this implies a greater energetic input needed for heavier isotopologues to reach the transition state, and consequently a slower reaction rate.

Isotopic rate changes are most pronounced when the relative mass change is greatest, since the effect is related to vibrational frequencies of the affected bonds. For instance, changing a hydrogen atom (H) to its isotope deuterium (D) represents a 100 % increase in mass, whereas in replacing $^{12}C$ with $^{13}C$, the mass increases by only 8 percent. The rate of a reaction involving a C-H bond is typically 6-10 times faster than the corresponding C-D bond, whereas a $^{12}C$ reaction is only 4 percent faster than the corresponding $^{13}C$ reaction.

A primary kinetic isotope effect may be found when a bond to the isotope atom is being formed or broken. A secondary kinetic isotope effect is observed when no bond to the isotope atom in the reactant is broken or formed. Secondary kinetic isotope effects tend to be much smaller than primary kinetic isotope effects; however, secondary deuterium isotope effects can be as large as 1.4 per deuterium atom.

Step (a)

Step (a) concerns the electrolysis of water using electrical power generated at least in part from non-fossil energy.

Electrolysis of water is an environmentally friendly method for production of hydrogen because it uses renewable $H_2O$ and produces only pure oxygen as by-product. Additionally, water electrolysis utilizes direct current (DC) from sustainable energy resources, for example solar, wind, hydropower and biomass.

It is observed that by electrolysis of water, the deuterium atom content of the hydrogen is lower than in the hydrogen generated petrochemically, for example as contained in synthesis gas, in general $\leq$ 100 ppm, preferably in general $\leq$ 90 ppm, for example from 30 to 75 ppm. The deuterium atom content in electrolytically produced hydrogen may be as low as 10 ppm. The deuterium is mainly present in the form of D-H rather than $D_2$.

One suitable water electrolysis process is alkaline water electrolysis. Hydrogen production by alkaline water electrolysis is a well-established technology up to the megawatt range for a commercial level. In alkaline water electrolysis initially at the cathode side two water molecules of alkaline solution (KOH/NaOH) are reduced to one molecule of hydrogen

($H_2$) and two hydroxyl ions (OH-). The produced $H_2$ emanates from the cathode surface in gaseous form and the hydroxyl ions (OH-) migrate under the influence of the electrical field between anode and cathode through the porous diaphragm to the anode, where they are discharged to half a molecule of oxygen ($O_2$) and one molecule of water ($H_2O$). Alkaline electrolysis operates at lower temperatures such as 30-80°C with alkaline aqueous solution (KOH/NaOH) as the electrolyte, the concentration of the electrolyte being about 20% to 30 %. The diaphragm in the middle of the electrolysis cell separates the cathode and anode and also separates the produced gases from their respective electrodes, avoiding the mixing of the produced gases. However, alkaline electrolysis has negative aspects such as limited current densities (below 400 mA/cm$^2$), low operating pressure and low energy efficiency.

In one preferred embodiment of the inventive process, hydrogen is provided by polymer electrolyte membrane water electrolysis. Variants of polymer electrolyte membrane water electrolysis are proton exchange membrane water electrolysis (PEMWE) and anion exchange membrane water electrolysis (AEMWE).

PEM water electrolysis was developed to overcome the drawbacks of alkaline water electrolysis. PEM water electrolysis technology is similar to the PEM fuel cell technology, where solid polysulfonated membranes (Nafion®, fumapem®) are used as an electrolyte (proton conductor). These proton exchange membranes have many advantages such as low gas permeability, high proton conductivity (0.1 $\pm$ 0.02 S cm$^{-1}$), low thickness (20-300 $\mu$m), and allow high-pressure operation. In terms of sustainability and environmental impact, PEM water electrolysis is one of the most favorable methods for conversion of renewable energy to highly pure hydrogen. PEM water electrolysis has great advantages such as compact design, high current density (above 2 A cm$^{-2}$), high efficiency, fast response, operation at low temperatures (20-80°C) and production of ultrapure hydrogen. The state-of-the-art electrocatalysts for PEM water electrolysis are highly active noble metals such as Pt/Pd for the hydrogen evolution reaction (HER) at the cathode and $IrO_2/RuO_2$ for the oxygen evolution reaction (OER) at the anode.

One of the largest advantages of PEM water electrolysis is its ability to operate at high current densities. This can result in reduced operational costs, especially for systems coupled with very dynamic energy sources such as wind and solar power, where sudden spikes in energy output would otherwise result in uncaptured energy. The polymer electrolyte allows the PEM water electrolyzer to operate with a very thin membrane (ca. 100-200 $\mu$m) while still allowing high operation pressure, resulting in low ohmic losses, primarily caused by the conduction of protons across the membrane (0.1 S/cm), and a compressed hydrogen output.

The PEM water electrolyzer utilizes a solid polymer electrolyte (SPE) to conduct protons from the anode to the cathode while insulating the electrodes electrically. Under standard conditions the enthalpy required for the formation of water is 285.9 kJ/mol. One portion of the required energy for a sustained electrolysis reaction is supplied by thermal energy and the remainder is supplied through electrical energy.

The half reaction taking place on the anode side of a PEM water electrolyzer is commonly referred to as the Oxygen Evolution Reaction (OER). Here the liquid water reactant is supplied to a catalyst where it is oxidized to oxygen, protons and electrons.

The half reaction taking place on the cathode side of a PEM water electrolyzer is commonly referred to as the Hydrogen Evolution Reaction (HER). Here the protons that have moved through the membrane are reduced to gaseous hydrogen.

PEMs can be made from either pure polymer membranes or from composite membranes, where other materials are embedded in a polymer matrix. One of the most common and commercially available PEM materials is the fluoropolymer PFSA, or Nafion®, a DuPont product. While Nafion® is an ionomer with a perfluorinated backbone like Teflon, there are many other structural motifs used to make ionomers for proton-exchange membranes. Many use polyaromatic polymers, while others use partially fluorinated polymers.

An overview over hydrogen production by PEM water electrolysis is given in S. Kumar and V. Himabindu, Material Science for Energy Technologies 2 (2019), pp. 4442 - 4454.

An overview over hydrogen production by anion exchange membrane water electrolysis is given in H. A. Miller et al., Sustainable Energy Fuels, 2020, 4, pp. 2114 - 2133.

K. Harada et al., International Journal of Hydrogen Energy 45 (2020), pp. 31389 - 31 395 report a deuterium depletion by a factor from 2 to 3 in polymer electrolyte membrane water electrolysis. The separation factor $\beta$

$$\beta = ([H]/[D])_{gas} / ([H]/[D])_{liquid}$$

where "gas" is the evolved gas and "liquid" is water before the electrolysis was found to be between 2 and 3 at current densities of from 1.0 to 2.0 A cm$^{-2}$, corresponding to a stoichiometric number A of between 4 and 9 at the given water mass flow in the anode. The stoichiometric number A is defined as follows:

$$\lambda = V \times \rho / (J/2F \times 60 \times M_{H2O})$$

where V (mL min$^{-1}$) is the water mass flow in the anode, F is the Faraday constant, J is electrolysis current (A), $\rho$ is the density of water (g mL$^{-1}$) and M$_{H2O}$ (g mol$^{-1}$) is the molar weight of water. A stoichiometric number A of 10 means that 10 times the amount of fresh water than can be theoretically consumed by electrolysis at the given electrolysis current is supplied to the anode.

H. Sato et al., International Journal of Hydrogen Energy 46 (2021), pp. 33 689 - 33 695, report for anion exchange membrane water electrolysis that deuterium concentration in the evolving hydrogen gas is diluted by approximately 1/5 against the feed water, at A = 4.

Hence, deuterium in the evolving hydrogen gas can easily be depleted by a factor of from 2 to 5 with regard to feed water in polymer electrolyte membrane water electrolysis. Depending on the electrolysis conditions (water flow, current density), even higher depletion factors are possible. Since the average deuterium content of water is about 150 ppm, based on the total hydrogen content, hydrogen provided in step (a) of the inventive process may have a deuterium content of from 30 to 75 ppm, based on the total hydrogen content, or even lower.

The electrical power is generated at least in part from non-fossil, renewable resources. In other words, part of the electrical power can still be produced from fossil fuels, preferably from natural gas, since combustion of natural gas causes much lower carbon dioxide emission per Megajoule of electrical energy produced than combustion of coal. However, the portion of electrical energy produced from fossil fuels should be as low as possible, preferably $\leq$ 50%, preferably $\leq$ 30%, most preferably $\leq$ 20%.

The electrical power from non-fossil resources used in water electrolysis according to the invention can be generated by nuclear energy. Nuclear energy is considered renewable by the European Commission, as long as certain preconditions (i. e. safe long-term storage of nuclear waste) are fulfilled.

The electrical power from non-fossil resources used in water electrolysis according to the invention is preferably generated from wind power, solar energy, biomass, hydropower and geothermal energy.

In one preferred embodiment of the inventive process, the electrical power used in water electrolysis is generated from hydropower. There are many forms of hydropower. Traditionally, hydroelectric power comes from constructing large hydroelectric dams and reservoirs. Small hydro systems are hydroelectric power installations that typically produce up to 50 MW of power. They are often used on small rivers or as a low-impact development on larger rivers. Run-of-the-river hydroelectricity plants derive energy from rivers without the creation of a large reservoir. The water is typically conveyed along the side of the river valley (using channels, pipes and/or tunnels) until it is high above the valley floor, whereupon it can be allowed to fall through a penstock to drive a turbine.

Wave power, which captures the energy of ocean surface waves, and tidal power, converting the energy of tides, are two forms of hydropower with future potential.

In one further preferred embodiment of the inventive process, the electrical power used in electrolysis is generated at least in part from geothermal energy. Geothermal energy is the heat that comes from the sub-surface of the earth. It is contained in the rocks and fluids beneath the earth's crust and can be found as far down to the earth's hot molten rock, magma.

To produce power from geothermal energy, wells are dug a mile deep into underground reservoirs to access the steam and hot water there, which can then be used to drive turbines connected to electricity generators. There are three types of geothermal power plants: dry steam, flash and binary. Dry steam is the oldest form of geothermal technology and takes steam out of the ground and uses it to directly drive a turbine. Flash plants use high-pressure hot water into cool, low-pressure water whilst binary plants pass hot water through a secondary liquid with a lower boiling point, which turns to vapor to drive the turbine.

In one further preferred embodiment of the inventive process, the electrical power used in water electrolysis is generated from wind power. Wind power can be used to run wind turbines. Modern utility-scale wind turbines range from around 600 kW to 9 MW of rated power. The power available from the wind is a function of the cube of the wind speed, so as wind speed increases, power output increases up to the maximum output for the particular turbine. Areas where winds are stronger and more constant, such as offshore and high-altitude sites, are preferred locations for wind farms.

In one further preferred embodiment of the inventive process, the electrical power used in water electrolysis is generated from solar power, particularly preferred from photovoltaic systems. A photovoltaic system converts light into electrical direct current (DC) by taking advantage of the photoelectric effect. Concentrated solar power (CSP) systems use lenses or mirrors and tracking systems to focus a large area of sunlight into a small beam. CSP-Stirling has by far the highest efficiency among all solar energy technologies.

In one further preferred embodiment of the inventive process, the electrical power used in water electrolysis is generated from biomass. Biomass is biological material derived from living, or recently living organisms. It most often refers to plants or plant-derived materials which are specifically called lignocellulosic biomass. As an energy source, biomass can either be used directly via combustion to produce heat or electricity, or indirectly after converting it to various forms of biofuel. Conversion of biomass to biofuel can be achieved by different methods which are broadly classified into: thermal, chemical, and biochemical methods. Wood was the largest biomass energy source as of 2012; examples include forest residues - such as dead trees, branches and tree stumps -, yard clippings, wood chips and even municipal solid waste. Industrial biomass can be grown from numerous types of plants, including miscanthus, switchgrass, hemp, corn, poplar, willow, sorghum, sugarcane, bamboo, and a variety of tree species, ranging from eucalyptus to oil palm (palm oil).

Plant energy is produced by crops specifically grown for use as fuel that offer high biomass output per hectare with low input energy. The grain can be used for liquid transportation fuels while the straw can be burned to produce heat or electricity. Biomass can be converted to other usable forms of energy such as methane gas or transportation fuels such as ethanol and biodiesel. Rotting garbage, and agricultural and human waste, all release methane gas - also called landfill gas or biogas. Crops, such as corn and sugarcane, can be fermented to produce the transportation fuel, ethanol. Biodiesel, another transportation fuel, can be produced from left-over food products such as vegetable oils and animal fats.

Step (b)

Step (b) concerns the reaction of the hydrogen from step (a) with nitrogen to form ammonia.

The reaction of step (b) preferably follows the Haber-Bosch process.

The catalysts usually used in the Haber-Bosch process generally fall into one of two categories, fused-iron and supported metallic catalysts. Fused-iron catalysts are derived from iron oxides, of which there are three possibilities: $Fe_2O_3$, $Fe_3O_4$, and $Fe_{1-x}O$, which are known as hematite, magnetite, and wüstite, respectively. Industrially, these iron catalysts will be multipromoted with promoters such as $K_2O$, BaO, KOH, CaO, MgO and $Al_2O_3$ are present in small quantities of a few weight percent. Supported metallic catalysts are catalysts made up of a metallic catalyst material, normally ruthenium or cobalt for the ammonia synthesis reaction, present on the surface of a support material, normally activated carbon or a metal oxide. Commonly the weight percentage of the metallic catalyst is around 2-10%.

Other catalysts which may be used are nickel, and nitride catalyst systems or electride, hydride, nitride, oxynitride hydride promoted Ru, Fe, Co, and Ni catalysts.

The catalysts mentioned above can be used for both conventional centralized large-scale Haber-Bosch ammonia synthesis plants and distributed small-scale ammonia production via the same process.

In one embodiment of the present invention, step (b) is performed at a pressure in the range of from 50 to 350 bar (abs), preferably 150 to 300 bar (abs).

In one embodiment of the present invention, step (b) is performed at a temperature in the range of from 300 to 600 °C, preferably 400 to 500 °C.

By performing step (b), ammonia is formed. The deuterium content of the ammonia is significantly lower than obtained by classical petrochemical routes. The deuterium content is in general ≤ 100 ppm, preferably in the range of from 10 to 95 ppm, more preferably in the range of from 15 to 90 ppm, most preferably in the range of from 20 to 80 ppm, especially in the range of from 30 to 75 ppm, based on the total hydrogen content.

The overall kinetic isotope effect is cumulative, since it will also be present in all subsequent production steps downstream the value chain.

Step (c)

In step (c), the ammonia from step (b) is reacted with a nitrile compound or hydrogen cyanide (I)

R-CN    (I)

in the presence of hydrogen from step (a) to form the corresponding amine (II)

$RCH_2-NH_2$    (II).

An overview of the process of hydrogenating nitrile compounds in the presence of ammonia has been given in Roose, P., Eller, K., Henkes, E., Rossbacher, R. and Hoke, H. (2015). Amines, Aliphatic, in Ullmann's Encyclopedia of Industrial Chemistry, https://doi.org/10.1002/14356007.a02_001.pub2;, and Roose, P. and Turcotte, M.G. (2016), Amines, lower aliphatic, in Kirk-Othmer Encyclopedia of Chemical Technology, John Wiley & Sons, Inc (Ed.). https://doi.org/10.1002/0471238961.1215230520211803.a01.pub3.

Additional information on hydrogenation of nitriles is given in C.Stock (2023) Hydrogenation and Dehydrogenation; Chapter 1.9.9 Hydrogenation of Nitriles; in Ullmann's Encyclopedia of Industrial Chemistry (https://doi.org/10.1002/14356007.a13_487.pub3).

The process for hydrogenating nitriles in the presence of ammonia to give primary amines uses a catalyst which contains active metal particles. The catalyst is composed of various active metals on an oxidic support, and either extruded or shaped into pellets or other more intricate shaped bodies. The active metals are selected from transition metals, preferably from Ni, Co, Cu, Ag, Au, Pd, Pt, Rh, Ru, Ir, Fe. Optionally, other metals can be added to moderate the activity and selectivity of the catalytically active metals, like Al, Mo, Sb, Bi, Sn, Mn, Cr. Other elements like rare earth metals, e.g., La or Y, and main group elements like Li, Na, K, Ca, Ba, Cs, P, S, or carbon may also be present and can enhance mechanical stability, selectivity or lifetime of the catalysts. Preferred are Ni, Co, Cu, Ag, Pd, Pt, Ru as active metals, and Sn, Mo, Mn, La, Li, Na, P as further admixtures. Supports are chosen from aluminum oxide, silicon oxide, zirconium oxide, titanium oxide, active carbon, preferably from aluminum oxide, silicon oxide, zirconium oxide or mixtures of these metal oxides. Preferably, when using a supported metal catalysts, the metal particles on the support are nanoparticles with a size of 3-30 nm as described in DE 10152135 (BASF AG). Optionally, particles of elemental metals and binders like cement can also be added to the catalyst to facilitate the shaping or prolonging the lifetime. Suitable catalysts are also so-called Raney-type catalysts or sponge catalysts which are made by leaching a metal-aluminum alloy (typically Ni or Co-Al alloy) with NaOH. The resulting macroporous catalysts are highly active and can be used as wet slurries in suspension catalysis or shaped with appropriate binders into fixed bed catalysts. Such catalysts can also be produced in a foam-like structure or hollow spheres depending on whether the alloy is applied to an appropriate support before leaching. The support may be removed at any stage of this process leaving a highly active shaped sponge catalyst. The Raney-type catalysts can be promoted by various metals in minor amounts, e.g., Cr, Fe, Mo and the like.

The process can be carried out in continuous mode or in batch- or fed-batch mode. Preference is given to continuous or semi-continuous (fed-batch) mode. Reactors can be for example tubular reactors, multi-tube reactors, shaft reactors, shell-tube reactors, continuous stirred tank reactors, cascades of such reactors, jet-loop reactors, bubble columns, gas lift reactors, stirred tank reactors or combinations thereof. The process is preferably carried out in continuous mode, and the catalyst is preferably arranged as a fixed bed in the reactor. Flow through the fixed bed of catalyst can be either from the top or from the bottom. For so-called Strecker nitriles obtained by the reaction of hydrogen cyanide with ammonia, other amines like ethylenediamine and formaldehyde, the use of slurry process with excess ammonia may be advantageous, as described in WO2014/131620, to BASF SE. In addition to the liquid phase, a gas phase may be present. The reactants (nitrile plus ammonia) are passed simultaneously, including hydrogen, over the catalyst at pressures of generally 5 to 50 MPa (50-500 bar), preferably 7 to 35 MPa, more preferably 10 to 19 MPa, and temperatures of generally 30 to 350°C, particularly 40 to 300°C, preferably 50 to 200°C, more preferably 50 to 180°C, in particular 50 to 150°C. The catalyst hourly space velocity is generally in the range from 0.05 to 5 kg, preferably 0.1 to 2 kg and more preferably 0.2 to 1.5 kg of nitrile per liter of catalyst (bed volume) and hour. The molar ratio of hydrogen to nitrile used is generally from 2:1 to 40:1, preferably from 2.01:1 to 10:1. The hydrogen can be returned as recycle gas to the reaction.

Ammonia is generally added in molar ratios to the nitrile group of from 0.5:1 to 500:1, preferably from 2:1 to 40:1, very preferably from 2:1 to 20:1. Ammonia prevents the condensation of the intermediate imine with the product amine which would result in formation of a secondary amine. Ammonia thus increases the yield or selectivity of the process towards the primary amine. In addition to ammonia, other additives can be used in order to suppress formation of the secondary amine as described in EP 3 061 743 A1 (BASF SE). Such additives are compounds of the alkali metals Li, Na, K, Rb, Cs or alkaline earth metals Mg, Ca, Sr and Ba compounds or compounds of rare earths metals La, Ce, Pr and Nd. Preferred compounds are compounds of Li, Na, K, Cs, Mg, Ca, La and Ce. Most preferred are compound of Li, Na, K. Preferably, the compounds are salts like hydroxides, oxides, acetates, nitrates and so on; most preferably they are chosen from Li, Na, and K hydroxides and employed as aqueous solutions.

The reaction can also be carried out in the presence of water. However, the water content should be not more than 10% by weight, preferably less than 5% by weight, particularly preferably less than 3% by weight, based on the mass of the liquid used, in order to very largely avoid leaching and/or washing off of the compounds of the alkali metals, alkaline earth metals and/or rare earth metals.

If appropriate, the reactants can be diluted with a suitable solvent such as alcohols (methanol, ethanol, isopropanol, high-boiling oxygenates), hydrocarbons like hexane and cyclohexane, aromatics like toluene and xylene, ethers like tetrahydrofuran, methyl-tetrahydrofuran, dioxane, or ethylene glycol dimethyl ether and amides like N-methylpyrrolidone, dimethylformide and dimethylacetamide. It is appropriate to heat the reactants before they are fed into the reaction vessel, preferably to the reaction temperature. It is possible to use higher temperatures and higher overall pressures and catalyst hourly space velocities. The pressure in the reaction vessel, which results from the sum of the partial pressures of ammonia, nitrile, and of the reaction products formed and, if appropriate, of the solvent used at the temperatures specified, is appropriately increased by injecting hydrogen up to the desired reaction pressure. The excess ammonia can be circulated together with the hydrogen. In order to remove the heat of reaction from the catalyst bed, it is advantageous to recirculate part of the crude product obtained in the gas-liquid separator which may still contain some ammonia. It is advantageous to work with high cross-sectional loadings of reactants, i.e., to maintain a suitably high amount of reactant flow in relation to the diameter of the tubular reactor. Suitable cross-sectional loadings are in the range of 5-50 kg/m$^2$/s as described in US 2012/0245390A1 (BASF SE). Such cross-sectional loadings ensure sufficient mass transfer from the gas phase (hydrogen) into the liquid phase and to the catalyst surface. In the case of slurry processes, this mass transfer is brought about by sufficiently high stirring speed, mixing with a pump or in a jet. After the reaction effluent has been decompressed appropriately, the excess hydrogen and ammonia present are removed and the resulting crude reaction product is purified, for example by a fractional rectification. The excess ammonia and the hydrogen are advantageously returned into the reaction zone. The same applies to any incompletely converted nitrile. The activity and/or selectivity of the catalysts according to the invention can decrease with an increasing period of operation. The catalyst can be regenerated by treatment with a liquid, optionally in the presence of hydrogen. The treatment of the catalyst with a liquid should lead to any adhering compounds which block active sites of the catalyst being dissolved. The treatment of the catalyst with a liquid can be carried out by stirring the catalyst in a liquid or by washing the catalyst with the liquid; after the treatment is complete, the liquid can be separated off together with the dissolved impurities from the catalyst by filtration or decantation.

Suitable liquids are as a rule the product of the hydrogenation, water or an organic solvent, preferably ethers, alcohols or amides.

Primary amines are compounds which have at least one primary amino group ($-NH_2$). Primary diamines have two primary amino groups. Primary triamines have three primary amino groups. Primary polyamines have more than three primary amino groups. Primary amines are valuable products with a large number of different uses, for example as solvents, stabilizers, for the synthesis of chelating agents, as starting materials for producing synthetic resins, inhibitors, interface-active substances, intermediates in the manufacture of fuel additives (U.S. Pat. No. 3,275,554 A, DE 2125039A and DE36 11 230 A), surfactants, drugs and crop protection agents, hardeners for epoxy resins, catalysts for polyurethanes, intermediates for producing quaternary ammonium compounds, plasticizers, corrosion inhibitors, synthetic resins, ion exchangers, textile auxiliaries, dyes, vulcanization accelerators and/or emulsifiers. Primary diamines and triamines are valuable products with a large number of different uses, for example as solvents, stabilizers, for the synthesis of chelating agents, as starting materials for producing synthetic resins and fibers (Nylon 6,6, Nylon 4, 12), drugs, inhibitors, corrosion protectants, polyurethanes, as hardeners for epoxy resins, and interface-active substances.

Preferred nitriles to be hydrogenated according to the present invention are:

a) cyanoalkylated amines, such as dimethylaminopropionitrile, bis(cyanoethyl)propanediamine, monocyanoethylpropanediamine, bis(cyanoethyl)butanediamine, cyanoethylbutanediamine and bis(cyanoethyl)methylamine;
b) ethernitriles obtained by reaction of alcohols with acrylonitrile, such as methoxypropionitrile;
c) $C_2$-$C_{18}$-ether propionitriles obtained by reaction of the corresponding alcohol with acrylonitrile,
d) Cyanoethylated long-chain etheramines,
e) bis(cyanoethyl)diethylene glycol and monocyanoethyldiethylene glycol, bis(cyanoethyl)butanediol and monocyanoethylbutanediol;
f) reaction products of prussic acid (HCN) with alkylene oxides, such as ethylenoxide, with alkylchlorides, butadiene, acrylonitrile, formaldehyde, ammonia or amines such as ethylenediamine (so-called Strecker nitriles);
g) aliphatic and aromatic mono- di-, tri- and tetranitriles, such as propionitrile, succinodinitrile, adipodinitrile, suberodinitrile, 1,3,6-hexanetrinitrile, benzyl cyanide, ortho- and meta-xylylenedinitrile.

Preferred amines that can be obtained from the corresponding nitriles are aliphatic, heteroaliphatic (i. e. containing hetero atoms in the aliphatic chain), araliphatic, cycloaliphatic or heterocycloaliphatic amines or polyamines having 2 to 24 carbon atoms and 1 to 3 primary amino groups ($-NH_2$).

Important amines that can be obtained according to the present invention are 1,3-propan-diamine, (dimethylamino)propylamine, bis(dimethylaminopropyl)amine, 3-aminopropanol, ethylenediamine, diethylenetriamine, triethylenetetramine, methoxypropylamine, dioxadodecanediamine, aminopropyldiethylene glycol, dioxanonanediamine (obtained by amination of the hydrogenated mono addition product of diethyleneglycol to acrylonitrile), propylamine, dipropylamine, tripropylamine, trioxatridecanediamine, N-[3-(dodecyloxy)propyl]propylenediamine, N-dodecyl-1,3-propylenediamine, 3-(dodecyloxy)propylamine, aminopropylcaprolactam, benzylamine, (diethylamino)ethylamine, diethylaminoethylamine, dipropylenetriamine, tripropylenetetramine, isophoronediamine, monohexylamine, dihexylamine, trihexylamine, N(3-aminopropyl)imidazol, N,N-bis-(3-aminopropyl)methylamine, N,N-diethylaminopropylamine, aminopropylethylenediamine, N,N'-bis(aminopropyl)ethylenediamine, aminopropylbutanediamine, N,N'-bis(aminopropyl)butanediamine 1,4-butandiamine, hexamethylenediamine, N,N-dimethyldipropylentriamine, n-octylamine, octamethylenediamine, 4-aminomethyloctanediamine, orthophthalodiamine, 1,3-xylylenediamine and 2-phenylethylamine.

The inventive amine is characterized by a low molar share of deuterium in the $-CH_2NH_2$ group or groups formed in step (c), which is $\leq 100$ ppm, preferably in the range of from 10 to 95 ppm, more preferably in the range of from 15 to 90 ppm, most preferably in the range of from 20 to 80 ppm, especially 30 to 75 ppm, based on the total hydrogen content of said $-CH_2NH_2$ group or groups. Said amines are prepared by the process of the present invention comprising steps (a) to (c).

Said low deuterium molar share is introduced by step (a) of the process according to the present invention. Therefore, also the ammonia, prepared by reacting the hydrogen from step (a) with nitrogen in step (b) is characterized by this low deuterium molar share.

With the present invention environmentally friendly amines and an environmentally friendly process for making the same, wherein said process uses as little fossil energy as possible, are provided.

As mentioned above, it is important that the origin of the hydrogen and down-stream compounds obtained by clean energy can be tracked in a reliable way.

Today, the majority of hydrogen is produced from fossil fuels by steam reforming of natural gas and other light hydrocarbons, partial oxidation of heavier hydrocarbons, and coal gasification. According to the invention, hydrogen obtained by electrolysis is obtained by using non-fossil energy sources. It is expected that the electrification (power generation) of fossil sources will be fully replaced by the generation of power by non-fossil resources in the near future. The downstream products based on hydrogen, preferably amines and ammonia based on hydrogen obtained by electrolysis and hydrogen itself, can be distinguished by its deuterium molar share from amines, ammonia and hydrogen prepared by processes based on fossil energy, i.e. made by petrochemical processes.

The present invention therefore relates to the use of the molar share of deuterium in downstream compounds based on hydrogen for tracing the origin, especially the energetic origin, of the hydrogen bound in the downstream compounds based on hydrogen, wherein the downstream compounds are amines.

The present invention further relates to a process for tracing the origin, especially the energetic origin, of hydrogen bound in downstream compounds based on hydrogen by determining the molar share of deuterium in said downstream compounds based on hydrogen, wherein the downstream compounds are amines.

Tracing is in the meaning of the present invention synonymous with tracking.

The origin is in the meaning of the present invention the preparation method of the hydrogen employed, especially electrolysis and/or the energetic origin, i.e. non-fossil energy sources. As mentioned above, it is expected that the electrification (power generation) of fossil sources will be fully replaced by the generation of power by non-fossil resources in the near future. Hydrogen made by electrolysis is in this case hydrogen of non-fossil origin. Examples for non-fossil power sources are mentioned above.

The inventive process for tracing the origin, especially the energetic origin, of hydrogen and downstream compounds mentioned above may be employed as a single tracing (tracking) method or in combination with further tracing (tracking) methods.

Suitable deuterium molar shares of the amines are mentioned in the present application.

Furthermore, the products according to the process described herein can be converted to a product as described on page 18, line 29 to page 26, line 29 of European patent application No. EP24164893.0.

Examples

I Hydrogen Production

Experimental Setup and Method: Electrolysis cell design and Hydrogen production conditions

1) Polymer Electrolyte Membrane / Proton Exchange Membrane Electrolysis (PEM)

Electrolysis Cell: Water electrolysis was conducted with a circular commercial PEM electrolysis cell (model ZE 200, Sylatech Analysetechnik GmbH, 0.007 m$^2$ active area). The cell stack was sealed with O-rings and wrapped with heat insulation fabric for isothermal operating conditions. A Nafion® 117 standard membrane (supplier DuPont, dry thickness 180 microns) was assembled by HIAT GmbH with catalytic active coating materials iridium (19 g/m$^2$) and platinum (8 g/m$^2$). Water distribution at the anode half-cell was realized with a titanium mesh. Before the polymer electrolyte membrane, a porous transport layer with sintered titanium fiber material is used for controlled flow while a porous graphite plate was situated on the cathode-side.

Experimental Conditions: Water with controlled temperature was supplied at constant flow of 9.5 g/h to the anode compartment. The cell pressure on cathode and anode side was controlled with PC valves. Experimental conditions such as temperature and pressure settings see table. The evolved hydrogen and oxygen gas in the half-cells was separated in a two-step separator setup with an intermediate condenser cooled with 20°C cooling water. Water condensate flowed back to the separator tank. Anodic cell-water was re-cycled, whereas on the cathode the separated water was drained. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

2) Alkaline Electrolysis Cell (AEC)

Electrolysis Cell: Alkaline water electrolysis was conducted with a circular AEC electrolysis cell (model Electro MP Cell, supplier ElectroCell Europe A/S, 0.01 m$^2$ electrode area). As electrodes Nickel 2.4068 material was used and separated by a commercial standard Zirfon Perl UTP 500 membrane (open mesh polyphenylene sulfide fabric symmetrically coated with a mixture polymer / zirconium oxide; thickness 500 microns; 0.023 m$^2$ active area; supplier Agfa-Gevaert N.V.) in zero-gap cell configuration.

Experimental Conditions: Alkaline water (32 wt% potassium hydroxide, technical standard grade) with controlled temperature was supplied at constant flow of 27.8 kg/h to the anode and cathode compartment. The cell pressure on cathode and anode side was equalized via PC valve control. Experimental conditions such as temperature and pressure settings see table. The evolved hydrogen and oxygen gas in the half-cells was separated in a two-step separator setup with an intermediate condenser cooled with 20°C cooling water. Water condensate flowed back to the separator tank. Anodic and cathodic cell-water was re-cycled. Remaining gas moisture is separated by desiccant dryers, ensuring complete separation of moisture in the electrolysis gas flow. Small gas samples of the dehydrated cathodic hydrogen gas were taken from the continous flow with an auto-sampler at regular intervals to conduct hydrogen gas analytics described below.

3) Anion Exchange Membrane / Alkaline Electrolyte Membrane Electrolysis (AEM)

Electrolysis Cell: The AEM experiments were executed in a commercial, fully automated 2.4 kW EL 4.0 cell supplied by Enapter GmbH, 10117 Berlin and a 1 wt% potassium hydroxide (standard grade) electrolyte solution.
Test station: As recommended by the supplier the EL 4.0 electrolyzer is run with a 1 wt% potassium hydroxide (technical standard grade) solution, hydrogen production at operating conditions (experimental conditions such as temperature and pressure settings see table) was 480 l/h at approx. 400 ml water consumption. The evolved hydrogen gas was treated with desiccant dryers, ensuring complete separation of moisture in the electrolysis gas flow to yield < 0,03 wt% water in the hydrogen gas stream. Small gas samples of the dehydrated cathodic hydrogen gas were taken from the continuous flow with an auto-sampler at regular intervals to conduct hydrogen gas analytics described below.

4) Solid Oxide Electrolysis Cell (SOE)

Solid Oxide cell stacks from Elcogen- Elco Stack (Elcogen OY, Vantaa 01510 Finland), were used and a E3000 unit was operated in reverse mode at 700°C and 35A electrolyzing current; Anode functional composition due to the supplier is NiO/YSZ. Cathode is of LSC type [La(Sr)CoO3]: The principle is also described in Novel high-performance solid oxide fuel cells with bulk ionic conductance dominated thin-film electrolytes - ScienceDirect (www.sciencedirect.com/science/article/pii/S037877531201097X); D. Stover et al, Journal of Power Sources; Volume 218, 15 November 2012, Pages 157-162.
The hydrogen stream was used with no further purification/dryer. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.
The results of the hydrogen production are shown in Table 1.

Table 1

| Electrolyser type | Example I) PEM-electrolyser | Example II) PEM electrolyser | Example III) Alkaline electrolyser | Example IV) Alkaline electrolyser | Example V) AEM | Example VI) AEM | Example VII) Solid oxide |
|---|---|---|---|---|---|---|---|
| Water-type/deuterium content | VSMOW/155,76 ppm D | Selected* surface water/139 ppm D | VSMOV/155,76 ppm D | Selected* surface water/139ppm D | VSMOW/155,76 ppm D | Selected* surface water/139 ppm D | Selected* surface water/139 ppm D |
| Current density [A/cm$^2$] | 1.35 | 1.35 | 0.8 | 0.8 | 1.6 | 1.6 | 0.78 |
| Applied voltage [V] | 1.9 | 1.9 | 2.15 | 2.15 | 1.9 | 1.9 | 1.35 |
| Operating temperature [°C] | 68 | 68 | 81 | 81 | 45 | 45 | 710 |
| Cell pressure [bar] | 23 | 23 | 25 | 25 | 35 | 35 | 1.3 |
| Voltage efficiency [%] | 57 | 57 | 53 | 53 | 66 | 66 | 81 |
| Electrical efficiency [kWh/kg H$_2$] | 52 | 52 | 48.5 | 48.6 | 69 | 69 | 47 |
| Deuterium content in hydrogen [ppm] | 86 | 77 | 95 | 87 | 74 | 66 | 38 |
| * Isar river surface water (Munich) collected in winter season January 2021. | | | | | | | |

Experimental Setup and Method: "D content (Deuterium content) in samples"

The following method descriptions apply for determination of the molar share of deuterium based on the total hydrogen content (Deuterium content) of gas and liquid samples. The isotopic H/D-share analysis is based on mass spectroscopy. Two different methods are used: method A for gas samples and method B for liquid samples.

For determination of the "D content in gas and liquid samples" it is of crucial importance not to contaminate the samples e.g. with ambient humidity or other ambient components containing hydrogen or deuterium. Therefore gas-tight materials and sealings must be used with clean sample containers to avoid any cross-contamination. Therefore, before filling and sealing a sample container it must be flushed at least 20 times the sample container volume with the gas or liquid stream to be analyzed. The same is valid for the experimental setup of the gas sampler and mass spectrometer. Utmost care must be taken to avoid cross-contamination e.g. via condensation of humidity. The analytical setup from sampling to mass spectrometry is validated with known reference samples.

Method A) Gas samples

Total Deuterium from HD and $D_2$ in hydrogen gas samples was determined via ultra-high resolution quadrupole mass spectrometry using a Hiden DLS-20 (Hiden Analytical Ltd., Warrington, Cheshire, UK) analyzer setup. The general method setup is described in C.C. Klepper, T.M. Biewer, U. Kruezi, S. Vartanian, D. Douai, D.L. Hillis, C. Marcus, Extending helium partial pressure measurement technology to JET DTE2 and ITER; Rev. Sci. Instrum., 87 (11) (2016); doi: 10.1063/1.4963713. For the hydrogen gas samples the threshold ionization mass spectrometry mode (TIMS) was used as described in S. Davies, J.A. Rees, D.L. Seymour; Threshold ionization mass spectrometry (TIMS); A complementary quantitative technique to conventional mass resolved mass spectrometry; Vacuum, 101 (2014), pp. 416-422; doi: 10.1016/j.vacuum.2013.06.004. Sensitivity is +/-1 ppm.

Method B) Liquid samples

Analysis of liquid samples (ammonia, alcohols and amines) was executed via isotope ratio monitoring gas chromatography/mass spectrometry (IRMS). Therefore a DELTA V PLUS CF-IRMS mass spectrometer was used. This mass spectrometer with magnetic sector with continuous flux DELTA V PLUS CF-IRMS is used to measure the isotopic ratio of D/H.

Measurement of D/H in a continuous He-flow mode needs the complete removal of low energy 4 He+ ions from the HD+ ion beam at m/z 3). The method is described in RAPID COMMUNICATIONS IN MASS SPECTROMETRY Rapid Commun. Mass Spectrom. 13, 1226-1230 (1999), W.A. Brandt et al. Sensitivity is within +/-3 ppm.

Method C) Analysis of isotope ratios in starting materials and products

Analysis of isotope ratios in starting materials and products was furthermore executed via SNIF-NMR related methods ("site-specific natural isotope fractionation studied by nuclear magnetic resonance") using high resolution $^2$H-NMR (Bruker Avance NEO 600 MHz NMR Spectrometer and Bruker Avance III HD 700 MHz NMR Spectrometer both equipped with TCI probes).

Principles of this technology are described in Gérard J. Martin, Serge Akoka, and Maryvonne L. Martin; SNIF-NMR Part 1: Principles; in Graham A. Webb (ed.), Modern Magnetic Resonance, Springer (2008) pp 1651-1658 as well as Maryvonne Martin, Benli Zhang, and Gérard J. Martin; SNIF-NMR Part 2: Isotope Ratios as Tracers of Chemical and Biochemical Mechanistic Pathways; in Graham A. Webb (ed.), Modern Magnetic Resonance, Springer (2008) pp 1659-1667 and Gérard J. Martin, Maryvonne L. Martin and Gérald Remaud; SNIF-NMR Part 3: From Mechanistic Affiliation to Origin Inference; in Graham A. Webb (ed.), Modern Magnetic Resonance, Springer (2008) pp 1669-1680.

II Production of Amines

Amines are prepared with catalytic synthesis processes in industrial scale as described above.

By using non-fossil-based energy for electrolytic hydrogen in both the ammonia and the hydrogen synthesis for the reductive amination of aldehydes and ketones, a substantial fraction of the hydrogen is originating from the electrolytic hydrogen with a low deuterium content. Especially for ammonia this is a big difference since the fossil-based production process relies on synthesis gas made from fossil natural gas and additionally large quantities of steam. This steam reforming step is not required for the non-fossil ammonia route based on pure hydrogen and nitrogen.

In all of the large-scale commercial production processes in question using non-fossil hydrogen no significant additional amounts of hydrogen-species are introduced in the "input/output" process material balance. This is required in order to minimize output of undesired wastewater or other liquid and gaseous emission streams and to ensure high yields with purity according to the product specification.

In case water is used as processing aid for stripping/scrubbing/cleaning or quenching/condensing of process-internal streams in e.g. the ammonia or amines synthesis steps this water is always run in an almost completely closed cycle with minimum purge ensuring insignificant cross-contamination with deuterium.

Examples

NI denotes volume in liters at standard conditions

Example 1: Isophorone diamine from Isophorone (general procedure)

The process for preparing isophorone diamine (= 3-aminomethyl-3,5,5-trimethyl-cyclohexylamine) from isophorone nitrile (3-Cyano-3,5,5-trimethyl-cyclohexanone) is carried out in two separate reaction spaces. In the first reaction space, 3-cyano-3,5,5-trimethyl-cyclohexanone is reacted with excess ammonia in the presence of an acidic metal oxide catalyst at a temperature of from 20 to 150°C and at a pressure of from 15 bis 500 bar. The obtained reaction products are hydrogenated with hydrogen in the presence of excess ammonia on a cobalt, nickel, ruthenium or other precious metal containing catalyst, optionally with basic components or on a neutral or basic carrier, at temperatures of from 60 to 150 °C and a pressure of from 60 to 300 bar.

Example 2: Isophorone diamine from Isophorone nitrile

A vertical tube reactor (diameter: 16 mm; filling height: 50 cm; oil-heated double jacket) was filled with 176.7 g (100 ml) of a basic cobalt full-contact (CoO with 5 % $Mn_2O_3$ and 1.4 % $Na_2O$) in the form of 1 to 1,5 mm split. For the reduction of the catalyst, the temperature was raised stepwise within 23 h from 100 to 330 °C and held for 30 h at 330°C while flowing 150 NI/h hydrogen through the reactor at 100 bar. Through a tube reactor (diameter: 16 mm, filling height: 50 cm, oil-heated double jacket) filled with 70.0 g (100 ml) of alumina in in the form of 1,5 mm-strands and mounted upstream from the hydrogenation reactor, 13.8 g molten isophorone nitrile (purity 99.0 %) and 303.0 g liquid ammonia were hourly pumped at 250 bar and at a temperature of 80°C from bottom to top. Then 60 NI/h (2.7 mol) of hydrogen were fed together with the output from the upstream-mounted imination reactor at a pressure of 250 bar and a temperature of 130 °C from bottom to top through the hydrogenation reactor. After depressurizing to normal pressure, ammonia was distilled off. The product mixture of the hydrogenation contained, according to GC analysis, 97.7 % isophorone diamine und 0.3 % 1,3,3-trimethyl-6-azabicyclo[3.2.1]octane, corresponding to a diamine-yield of 98.7%.

Example 3: Isophorone diamine from Isophorone nitrile

A vertical tube reactor (diameter: 16 mm, filling height: 100 cm, oil-heated double jacket) was filled with 354 g (200 ml) of a basic cobalt full-contact (CoO with 5 % $Mn_2O_3$ and 1.4 % $Na_2O$) in the form of 1 to 1.5 mm split. For the reduction of the catalyst, the temperature war raised stepwise within 23 h from 100 to 330 °C and held for 30 h at 330°C while flowing 150 NI/h hydrogen through the reactor at 100 bar. Through a tube reactor (diameter: 16 mm; filling height: 20 cm; oil-heated double jacket) filled with 25.4 g (40 ml) of $TiO_2$ (Anatas) in in the form of 1,5 mm-strands and mounted upstream of the hydrogenation reactor, 40 g molten isophorone nitrile (purity 99.0 %) and 102 g liquid ammonia were hourly pumped at 250 bar and at a temperature of 110°C from bottom to top. Then 100 NI/h (2.7 mol) of hydrogen were fed together with the output from the upstream-mounted imination reactor at a pressure of 250 bar and a temperature of 110 °C from bottom to top through the hydrogenation reactor. After depressurizing to normal pressure, ammonia was distilled off. The hydrogenation product mixture contained, according to GC analysis, 97.5 % isophorone diamine. The output was collected over 73 h and separated by rectification in a 30 cm packed fractionating column (3 mm glass rings). 2864 g of isophoronediamine were obtained, corresponding to a yield of 96.2 % of the theory.

**Claims**

1. A process for the preparation of amines comprising the following steps:

(a) providing hydrogen with a molar share of deuterium $\leq$ 100 ppm, based on the total hydrogen content, by electrolysis of water using electrical power generated at least in part from non-fossil energy,
(b) reacting the hydrogen from step (a) with nitrogen to form ammonia,
(c) reacting the ammonia from step (b) with a nitrile compound or hydrogen cyanide (I)

$$R\text{-}CN \qquad (I)$$

in the presence of hydrogen from step (a) to form the corresponding amine (II)

$$RCH_2\text{-}NH_2 \qquad (II).$$

2. The process of claim 1, wherein the electrical power is generated from wind power, solar energy, biomass, hydropower and geothermal energy.

3. The process according to claim 1 or 2, wherein hydrogen is provided by polymer electrolyte membrane water electrolysis.

4. The process according to claim 3, wherein hydrogen is provided by proton exchange membrane water electrolysis (PEMWE) or anion exchange membrane water electrolysis (AEMWE).

5. The process according to any one of claims 1 to 4, wherein the molar share of deuterium in the hydrogen provided in step (a) is in the range of from 10 to 95 ppm, preferably in the range of from 10 to 90 ppm, more preferably in the range of from 20 to 80 ppm, and most preferably in the range of from 30 to 75 ppm, based on the total hydrogen content.

6. The process according to any one of claims 1 to 5, wherein step (c) is carried out as heterogeneously or homogeneously catalyzed reaction.

7. The process according to any one of claims 1 to 6, wherein the nitrile compound (I) to be hydrogenated in step (c) is selected from:

a) cyanoalkylated amines, such as dimethylaminopropionitrile, bis(cyanoethyl)propanediamine, monocyanoethylpropanediamine, bis(cyanoethyl)butanediamine, cyanoethylbutanediamine and bis(cyanoethyl)methylamine;
b) ethernitriles obtained by reaction of alcohols with acrylonitrile, such as methoxypropionitrile;
c) $C_2$-$C_{18}$-ether propionitriles obtained by reaction of the corresponding alcohol with acrylonitrile;
d) cyanoethylated long-chain etheramines;
e) bis(cyanoethyl)diethylene glycol and monocyanoethyldiethylene glycol, bis(cyanoethyl)butanediol and monocyanoethylbutanediol;
f) reaction products of prussic acid (HCN) with alkylene oxides, such as ethylenoxide, with alkylchlorides, butadiene, acrylonitrile, formaldehyde, ammonia or amines, such as ethylenediamine;
g) aliphatic and aromatic mono- di-, tri- and tetranitriles, such as propionitrile, succinodinitrile, adipodinitrile, suberodinitrile, 1,3,6-hexanetrinitrile, benzyl cyanide, ortho- and meta-xylylenedinitrile.

8. Amines, obtainable in the process according to any one of claims 1 to 7.

9. Amines according to claim 8, wherein the molar share of deuterium in the -$CH_2NH_2$ group or groups formed in step (c) of the process according to any one of claims 1 to 6 is ≤ 100 ppm, preferably in the range of from 10 to 95 ppm, more preferably in the range of from 15 to 90 ppm, most preferably in the range of from 20 to 80 ppm, especially in the range of from 30 to 75 ppm, based on the total hydrogen content of said -$CH_2NH_2$ group or groups.

10. Amines according to claim 8 or 9, selected from the group consisting of aliphatic, heteroaliphatic, araliphatic, cycloaliphatic or heterocycloaliphatic amines or polyamines having 2 to 20 carbon atoms and 1 to 3 primary amino groups (-$NH_2$).

11. Amines according to claim 10, selected from the group consisting of 1,3-propan-diamine, (dimethylamino)propylamine, bis(dimethylaminopropyl)amine, 3-aminopropanol, ethylenediamine, diethylenetriamine, triethylenetetramine, methoxypropylamine, dioxadodecanediamine, dioxanonanediamine, aminopropyldiethylene glycol, propylamine, dipropylamine, tripropylamine, trioxatridecanediamine, *N-[3-(dodecyloxy)propyl]propylenediamine, N-dodecyl-1,3-propylenediamine, 3-(dodecyloxy)propylamine,* aminopropylcaprolactam, benzylamine, (diethylamino)ethylamine, diethylaminoethylamine, dipropylenetriamine, tripropylenetetramine, isophoronediamine, monohexylamine, dihexylamine, trihexylamine, N(3-aminopropyl)imidazol, N,N-bis-(3-aminopropyl)methylamine, N,N-diethylaminopropylamine, aminopropylethylenediamine, N,N'-bis(aminopropyl)ethylenediamine, aminopropylbutanediamine, N,N'-bis(aminopropyl)butanediamine 1,4-butanediamine, hexamethylenediamine, N,N-dimethyldipropylentriamine, n-octylamine, octamethylenediamine, 4-aminomethyloctanediamine, orthophthalodiamine, 1,3-xylylenediamine and 2-phenylethylamine.

12. The use of the molar share of deuterium in downstream compounds based on hydrogen for tracing the origin, especially the energetic origin, of hydrogen bound in the downstream compounds based on hydrogen, wherein the downstream compounds are amines.

13. The use according to claim 12, wherein the downstream compounds are amines according to any one of claims 8 to 11.

14. A process for tracing the origin, especially the energetic origin, of hydrogen bound in downstream compounds based on hydrogen by determining the molar share of deuterium in said downstream compounds based on hydrogen, wherein the downstream compounds are amines.

15. The process according to claim 14, wherein the downstream compounds are amines according to any one of claims 8 to 11.

Europäisches
Patentamt

European
Patent Office

Office européen
des brevets

# EUROPEAN SEARCH REPORT

**Application Number**

EP 24 20 7979

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/136097 A1 (SMAJLOVIC IVAN [RS]) 9 June 2011 (2011-06-09) * the whole document * | 11-15 | INV. C25B1/04 C25B15/08 C01B4/00 |
| A | FELLOWS S K ET AL: "Availability of large quantities of low-deuterium hydrogen, and possible uses", INTERNATIONAL JOURNAL OF HYDROGEN ENERGY, ELSEVIER, AMSTERDAM, NL, vol. 6, no. 1, 1 January 1981 (1981-01-01) , pages 67-71, XP025591252, ISSN: 0360-3199, DOI: 10.1016/0360-3199(81)90098-7 [retrieved on 1981-01-01] * the whole document * | 11-15 | C01C1/04 C07B59/00 C07C209/48 |
| Y | US 2020/148547 A1 (PAPILE CHRISTOPHER [US]) 14 May 2020 (2020-05-14) * the whole document * | 1-15 | |
| Y | US 2012/071694 A1 (EBERHARDT JAN [DE] ET AL) 22 March 2012 (2012-03-22) * the whole document * | 1-15 | TECHNICAL FIELDS SEARCHED (IPC) |
| Y | JP S51 48602 A (UBE INDUSTRIES) 26 April 1976 (1976-04-26) * the whole document * | 1-15 | C25B C07B C01C C01B C07C |
| A,P | WO 2023/213713 A1 (BASF SE [DE]) 9 November 2023 (2023-11-09) * claims 13, 14 * | 11-15 | |

The present search report has been drawn up for all claims

| Place of search | Date of completion of the search | Examiner |
|---|---|---|
| Munich | 5 March 2025 | Ritter, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 7979

This annex lists the patent family members relating to the patent documents cited in the above-mentioned European search report.
The members are as contained in the European Patent Office EDP file on
The European Patent Office is in no way liable for these particulars which are merely given for the purpose of information.

05-03-2025

| Patent document cited in search report | Publication date | Patent family member(s) | | Publication date |
|---|---|---|---|---|
| US 2011136097 A1 | 09-06-2011 | NONE | | |
| US 2020148547 A1 | 14-05-2020 | NONE | | |
| US 2012071694 A1 | 22-03-2012 | BR | PI0711043 A2 | 23-08-2011 |
| | | CN | 101443306 A | 27-05-2009 |
| | | CN | 102746186 A | 24-10-2012 |
| | | EP | 2018363 A1 | 28-01-2009 |
| | | ES | 2463686 T3 | 28-05-2014 |
| | | JP | 5227309 B2 | 03-07-2013 |
| | | JP | 2009536177 A | 08-10-2009 |
| | | US | 2009069590 A1 | 12-03-2009 |
| | | US | 2012071694 A1 | 22-03-2012 |
| | | WO | 2007128803 A1 | 15-11-2007 |
| JP S5148602 A | 26-04-1976 | JP | S5148602 A | 26-04-1976 |
| | | JP | S5810374 B2 | 25-02-1983 |
| WO 2023213713 A1 | 09-11-2023 | AU | 2023266064 A1 | 14-11-2024 |
| | | CN | 119156370 A | 17-12-2024 |
| | | EP | 4519241 A1 | 12-03-2025 |
| | | KR | 20250006960 A | 13-01-2025 |
| | | WO | 2023213713 A1 | 09-11-2023 |

## REFERENCES CITED IN THE DESCRIPTION

### Patent documents cited in the description

- DE 10152135
- WO 2014131620 A
- EP 3061743 A1
- US 20120245390 A1
- US 3275554 A
- DE 2125039 A
- DE 3611230 A
- EP 24164893

### Non-patent literature cited in the description

- **S. KUMAR** ; **V. HIMABINDU**. *Material Science for Energy Technologies*, 2019, vol. 2, 4442-4454
- **H. A. MILLER et al.** *Sustainable Energy Fuels*, 2020, vol. 4, 2114-2133
- **K. HARADA et al.** *International Journal of Hydrogen Energy*, 2020, vol. 45, 31389-31 395
- **H. SATO et al.** *International Journal of Hydrogen Energy*, 2021, vol. 46, 33 689-33 695
- **ROOSE, P.** ; **ELLER, K.** ; **HENKES, E.** ; **ROSSBA-CHER, R.** ; **HOKE, H.** Amines, Aliphatic. *Ullmann's Encyclopedia of Industrial Chemistry*, 2015, https://doi.org/10.1002/14356007.a02_001.pub2
- Amines, lower aliphatic. **ROOSE, P.** ; **TURCOTTE, M.G.** Kirk-Othmer Encyclopedia of Chemical Technology. 2016
- **C.STOCK**. Hydrogenation and Dehydrogenation. *Ullmann's Encyclopedia of Industrial Chemistry*, 2023, https://doi.org/10.1002/14356007.a13_487.pub3
- **D. STOVER et al.** *Journal of Power Sources*, 15 November 2012, vol. 218, 157-162
- **C.C. KLEPPER** ; **T.M. BIEWER** ; **U. KRUEZI** ; **S. VARTANIAN** ; **D. DOUAI** ; **D.L. HILLIS** ; **C. MARCUS**. Extending helium partial pressure measurement technology to JET DTE2 and ITER. *Rev. Sci. Instrum.*, 2016, vol. 87 (11)
- **S. DAVIES** ; **J.A. REES** ; **D.L. SEYMOUR**. Threshold ionization mass spectrometry (TIMS); A complementary quantitative technique to conventional mass resolved mass spectrometry. *Vacuum*, 2014, vol. 101, 416-422
- RAPID COMMUNICATIONS IN MASS SPECTROMETRY. *Rapid Commun. Mass Spectrom.*, 1999, vol. 13, 1226-1230
- SNIF-NMR Part 1: Principles. **GÉRARD J. MARTIN** ; **SERGE AKOKA** ; **MARYVONNE L. MARTIN**. Modern Magnetic Resonance. 2008, 1651-1658
- SNIF-NMR Part 2: Isotope Ratios as Tracers of Chemical and Biochemical Mechanistic Pathways. **MARYVONNE MARTIN** ; **BENLI ZHANG** ; **GÉRARD J. MARTIN**. Modern Magnetic Resonance. Springer, 2008, 1659-1667
- SNIF-NMR Part 3: From Mechanistic Affiliation to Origin Inference. **GÉRARD J. MARTIN** ; **MARYVONNE L. MARTIN** ; **GÉRALD REMAUD**. Modern Magnetic Resonance. Springer, 2008, 1669-1680